# EUROPEAN PATENT APPLICATION

(11) **EP 2 087 884 A2**
(43) Date of publication of application: **12.08.2009**
(21) Application number: 09157027.5
(22) Date of filing: 11.07.2008
(51) Int. Cl.: A61K 9/20, A61K 31/64

(54) **Extended Release Gliclazide Formulations**

(30) Priority: 13.07.2007 TR 200704897
(62) Divisional of application: 08104732.6
(71) Applicant: Sanovel Ilac Sanayi ve Ticaret A.S., 34398 Istanbul (TR)
(72) Inventor: Cifter, Ümit, 34398, Istanbul (TR); Türkyimaz, Ali, 34398, Istanbul (TR); Öner, Levent, Ankara (TR)
(74) Representative: Sevinç, Erkan

(57) **Abstract**

The present invention relates to an extended release (XL) pharmaceutical tablet composition for oral administration and methods of its manufacture. More particularly, the present invention relates to an extended release gliclazide formulation which does not increase the blood glucose level in human patients.

## Description

### Technical Aspect

The present invention relates to an extended release (XL) pharmaceutical tablet composition for oral administration and methods of its manufacture. More particularly, the present invention relates to an extended release gliclazide formulation which does not increase the blood glucose level in human patients.

The present invention further relates to an extended release pharmaceutical tablet composition which doesn't release more than %25 of the total amount of active substance in less than 2 hours, comprising therapeutically effective amount of gliclazide or a pharmaceutically acceptable salt thereof, mannitol as a binder and hydrophilic diluent in a ratio of 5 to 15 % (w/w) of total tablet composition that does not increase the blood glucose level in human patients, calcium hydrogen phosphate dihydrate or calcium hydrogen phosphate anhydrous as a diluent in a ratio of 6 to 50 % (w/w) of total tablet composition and hydroxypropylmethylcellulose (HPMC) as a rate controlling polymer.

### Background of the Invention

Diabetes is a metabolic abnormality mainly of glycometabolism, resulting from, insufficient insulin secretion, decreased sensitivity of target cells of insulin and so forth, and principally characterized by noticeable hyperglycemia. Serious detrimental complications arise in various organs and nerves such as retinopathy, nephropathy, neuropathy that are caused mainly by vascular lesion in case of the continuation of hyperglycemia for a long period of time.

For the time of being lots of anti-diabetic drugs such as sulfonylurea have been orally administered for the treatment of diabetics.

The sulfonylurea is a name of a group describing anti-diabetic drugs given by mouth in the treatment of type II diabetes mellitus. Diabetes mellitus is a group of disorders of carbohydrate metabolism in which the action of insulin is diminished or absent through altered secretion or decreased insulin activity; or a combination of both factors. Sulfonylureas are believed to stimulate the release of insulin from the pancreatic islet cells via receptors that are reported to be Adenosine 5'-triphosphate-sensitive potassium channels (K_{ATP}). Therefore sulfonylureas mainly acts by increasing endogenous insulin secretion providing an effective control on blood sugar levels in diabetics, in particular, type II diabetic patients who are unable to achieve control trough dietary.

The sulfonylureas are considered to be divided in to two categories: the first generation agents e.g., tolbutamide, chlorpropamide, tolazamide, acetohexamide and second generation agents e.g., glyburide (glibenclamide), glipizide, gliclazide.

One type of the second generation agent of sulfonylurea, gliclazide; N-(4-methylbenzenesulfonyl)-N'-(3-azabicyclo[3.3.0]-oct-3-yl)urea) having a anti-diabetic property at the doses usually administered to a human, is a known active ingredient amongst diabetics.

Gliclazide has hereto been administered orally in the form of tablets containing a dose of 80 mg. The usual average prescription is 1 tablet in 2 times a day, but may vary from 1 to 4 tablets per day in several administrations depending upon the severity of the diabetes.

The formulation of the active ingredient, gliclazide is firstly disclosed in the US patent no. 3,501,495 (SCIENCE UNION ET CIE. SOC.) 10.02.1966, describing the formula N-(4-methylbenzenesulfonyl)-N'-(3-azabicyclo[3.3.0]-oct-3-yl)urea) which has hypoglycemic properties and used per orally in the treatment of diabetes mellitus.

Immediate release formulation of sulfonylurea is elderly disclosed in several different patents.

Immediate release tablets, described in US patent no. 4,696,815, dated 08.06.1983 and formulated with sulfonylurea is based on an acidified and/or alkalized excipient, and an inert polar solvent, such as polyethylene glycol. These immediate release formulations are described as improving the dissolution of acidic, amphoteric or basic antidiabetic sulfonylurea compounds respectively. An analogous immediate release formulation with an acidified and/or alkalized excipient, an inert polar solvent and polyvynilpyrrolidone is also described by this US patent.

Other invention related to gliclazide is disclosed in US patent no. 6,733,782 (LES LABORATOIRES SERVIER) 01.02.1999, which concerns a core tablet for the controlled release of gliclazide which ensures continuous and constant release of the active principle by using hydroxypropyl methylcellulose (HPMC), unaffected by the dissolving medium pH variations, after oral administration. Main aim of this invention was to obtain an oral form that can be administered in a single daily dose, which also provide prolonged release. This US patent also teaches the use of glucose syrup, for example maltodextrin, along with cellulose polymers to achive a controlled and complete release of gliclazide from the formulation. Generally, it is undesirable to use glucose syrup or any other sugar based compound that has a high glysemic index as excipients in the preparation of formulations to be administered for the treatment of diabetes. The use of such excipients may increase the blood sugar levels in diabetic patients.

Dose dumping is one of the most important disadvantages of extended release dosage forms. The most important criteria of dose dumping under in-vitro conditions is the amount of the active substance released in early time point. This limit has to be within 20 to 30 % according to European Medicines Agency (EMEA) criteria. The released amount of active substance which is mentioned in US patent no 6,733,782 is 50% in 4 hours to 6 hours and this result is not a criteria for dose dumping.

The problem underlying the present invention was to provide an extended release pharmaceutical tablet composition which doesn't release more than %25 of the total amount of active substance in less than 2 hours.

Composition useful for reducing serum glucose levels by an oral controlled release system described in US patent no 6,703,045 (Council of Scientific & Industrial Research) 21.08.2001. Also, this patent describes a method of reducing serum glucose levels by an oral controlled release dosage form incorporating glipizide and the dosage form administered once a day will provide therapeutic levels of the drug throughout the day.

EP 1 741 435 A1 (Lotus Pharmaceutical Co. Ltd.) 29.04.2004 is related to a composition for a modified-release oral tablet, especially related to a composition comprising of a pharmaceutically effective amount of micronized active ingredient for lowering blood glucose level and a modified-release agent comprising of hydrophilic polymers, and a method for preparation of the novel composition for a modified-release oral tablet.

The PCT application WO 2006/061697 A1 (THEMIS LABORATORIES PRIVATE LIMITED) 06.12.2004, provides sustained release pharmaceutical compositions suitable for once a day administration and a process for preparing such compositions comprising sulfonylurea, polymer, disaccharide and/or monosaccharide (is selected from lactose, sucrose, maltose, galactose, trehalose maltitol, dextrose or their mixtures) exhibiting drug release profile substantially independent of pH of the dissolution medium in pH range 4 to 8.

The PCT application WO 2006/123213 A1 (RANBAXY LABORATORIES LIMITED) 18.05.2005, also relates to modified release formulations of gliclazide or salt thereof and processes for their preparation. The pharmaceutical formulation includes gliclazide or a salt thereof may have a D₉₀ range between less than about 70 µm to greater than about 18 µm or a D₉₀ less than about 18µm, one or more controlled release polymers, one or more binders and optionally one or more additional pharmaceutically acceptable excipients. The formulations provide a prolonged and substantially complete release of gliclazide or a salt thereof over a specified period of time.

Therefore, a novel and improved extended release pharmaceutical tablet composition which doesn't release more than %25 of the total amount of active substance in less than 2 hours, comprising therapeutically effective amount of gliclazide or a pharmaceutically acceptable salt thereof, mannitol as a binder and hydrophilic diluent in a ratio of 5 to 15 % (w/w) of total tablet composition that does not increase the blood glucose level in human patients, calcium hydrogen phosphate dihydrate or calcium hydrogen phosphate anhydrous as a diluent in a ratio of 6 to 50 % (w/w) of total tablet composition and hydroxypropylmethylcellulose (HPMC) as a rate controlling polymer is formulated.

### Detailed Description of the Invention

Diabetes mellitus is a complex chronic disorder of the carbohydrate, fat and protein metabolism that is primarily a result of a relative or complete lack of insulin secretion by the beta cells of the pancreas or of defects of the insulin receptors. The various forms of diabetes are divided in several categories, the two most frequent being juvenile-onset diabetes or Type I insulin dependent diabetes mellitus (IDDM) and adult-onset diabetes or Type II non-insulin dependent diabetes mellitus (NIDDM). Both diseases, even when correctly diagnosed and medicated, require life-long medication, good patient compliance, a careful diet and frequent medical observation to avoid potentially serious sequelae.

Uniformity and predictability of therapeutic levels of gliclazide and resulting blood sugar levels are considered to be desirable in the management of diabetes patients, and in particular, for the management of type II diabetic patients.

"Modified release dosage forms" are defined by the USP as those whose drug release characteristics of time course and/or location are chosen to accomplish therapeutic or convenience objectives not offered by conventional forms. An extended-release dosage form allows a twofold reduction in dosing frequency or increase in patient compliance or therapeutic performance. The USP considers that the terms controlled release; prolonged release and sustained release are interchangeable with extended release. Accordingly, the terms "modified-release", controlled-release", "prolonged-release", "extended-release", and "sustained-release" are used interchangeably herein.

In recent years there has been a large increase in the development and use of so called extended release formulations. Such formulations are designed to release a drug more or less slowly after ingestion compared to administering the drug per se or via an inert formulation. XL formulations can give improved therapeutic effects, reduced incidence of adverse effects and simplified dosing regimens. Generally XL tablets release the drug contained therein over several hours, typically more than 3 hours. Generally the drug will be released from the XL formulation in less than 30 hours. Several different types of extended release formulations are known, for example, Langer and Wise (Eds) "Medical applications of Controlled release", vols I and II, CRC Press Inc, Boca Raton, 1984; Robinson and Lee (Eds) "Controlled drag delivery - fundamentals and applications", Marcel Deldcer, NY, 1987; Bogentoft and Sjogren, in "Towards better safety of drugs and pharmaceutical products" (Ed: PCT/GBOI/03861 Braimer), Elsevier, 1980; Sandberg "Extended- release metoprolol", Thesis, Uppsala University, 1994. These known extended release formulations use a variety of mechanisms to control the release of an active substance in the formulation. The particular mechanism selected being largely dependent upon the therapeutic aims of the formulation. Examples of mechanisms exploited in extended release formulations include the control of dissolution, diffusion, swelling, osmotic pressure, complexation, ion-exchange or erosion of the tablet.

Polymers can be used to form a matrix in which an active substance is dispersed, the properties of the polymer are then utilised control the rate at which the active is released from the formulation. Such polymers include polysaccharides, especially the cellulose derivatives are hydroxypropyl methylcellulose (HPMC) and hydroxypropyl cellulose (HPC), see "Handbook of Pharmaceutical Excipients" (Ed: A Wade and P J Weller, Pharmaceutical Press, London 1994). The properties of the polymer when placed in water, especially in a physiological environment can be used to control the rate of release of an active substance from the XL formulation. For example, polymers such as HPMC often develop a gel-like outer layer that dissolves or erodes, thereby controlling the rate of water ingress into the XL formulation. The release of active substance from such XL formulations is believed to be controlled by a combination of several factors, including the properties of the active substance, the diffusion properties of the active substance or a solution thereof once dissolved, the penetration rate of the water into the polymer matrix, the swelling of the polymer matrix, and the rate of dissolution of the outer polymer gel layer.

However, many polymers used in XL formulations are sensitive to external factors and as a result are not sufficiently robust to be used in commercial XL formulations. Of particular importance is the sensitivity of the polymers to salt and surface active agents, e.g bile salts and lipids.

It is an object of the present invention to provide an extended release of a hypoglycemic drug which provides effective control of blood glucose levels in humans by using mannitol as a binder and hydrophyllic diluent in a ratio of 5 to 15% (w/w) of total tablet composition instead of maltodextrin (glucose syrup) and to obtain improved dissolution properties by using calcium hydrogen phosphate dihydrate or calcium hydrogen phosphate anhydrous in a ratio of 6 to 50 % (w/w) of total tablet composition with hydroxypropylmethylcellulose (HPMC) with a therapeutically effective amount of gliclazide or a pharmaceutically acceptable salt thereof.

Extended release sulfonylurea formulations with improved dissolution properties, are therefore a desirable addition to the medical treatment of diabetes, including type II diabetes.

In preferred embodiments, a novel and improved extended release solid oral dosage form of the present invention is a pharmaceutical tablet composition which doesn't release more than %25 of the total amount of active substance in less than 2 hours, comprising: (a) a therapeutically effective amount of gliclazide or a pharmaceutically acceptable salt thereof (b) mannitol as a binder and hydrophilic diluent in a ratio of 5 to 15% (w/w) of total tablet composition that does not increase the blood glucose level in human patients needing treatment for non-insulin dependent diabetes mellitus (NIDDM) (c) calcium hydrogen phosphate dihydrate or calcium hydrogen phosphate anhydrous as a diluent in a ratio of 6 to 50 % (w/w) of total tablet composition and (d) hydroxypropylmethylcellulose (HPMC) as a rate controlling polymer; said oral dosage form does not increase the blood glucose level.

The pharmaceutically acceptable rate controlling polymer can include but is not limited to a hydroxypropylmethylcellulose (HPMC), hydroxypropylcellulose (HPC), hydroxyethylcellulose (HEC), ethylcellulose (EC), methylcellulose (MC), sodium carboxymethylcellulose and celluloseacetatebutyrate or a mixture thereof.

The preferred polymer of the invention is HPMC which is used as a rate controlling polymer. In certain preferred embodiment the viscosity of HPMC is 100 cP or 4000 cP or combination of these. In this extended released pharmaceutical tablet composition, the rate controlling polymer is present in an amount of from 2 to 25% (w/w), calcium hydrogen phosphate dihydrate or calcium hydrogen phosphate anhydrous is present in an amount of from 6 to 50% (w/w) and the mannitol is present in an amount of from 5 to 15% (w/w).

In other preferred embodiments of this invention, the extended released pharmaceutical tablet composition can further comprising at least one additive ingredient wherein said additive ingredient is selected from the group comprising of flow agents and lubricants.

As mentioned above said flow agent is selected from the group consisting of colloidal silicon dioxide, silica, calcium silicate, magnesium trisilicate, sodium stearyl fumarate and talc, preferably the flow agent is colloidal silicon dioxide and it is present in an amount from 0.25 to 2.0%.

As mentioned above said lubricant is selected from the group consisting of magnesium stearate, calcium stearate, stearic acid, hydrogenated vegetable oils and vegetable based fatty acids, preferably the lubricant is magnesium stearate and it is present in an amount from 0.25 to 2.0%.

In prior art, it is known that gliclazide or a pharmaceutically acceptable salt thereof and HPMC are at least partially interdispersed. In certain embodiments, the gliclazide or a pharmaceutically acceptable salt thereof and HPMC comprise a homogenous mixture having a uniform dispersion, by adding calcium hydrogen phosphate dihydrate or calcium hydrogen phosphate anhydrous, a heterogeneous and pored matrix is formed. When the matrix tablet meets with the solution media, the hydrophilic mannitol is quickly dissolved and the active substance is diffused from the channels occurred in matrix. We have reached different dissolution profile results by using different amounts of mannitol.

We obtain suitable mannitol rates to make possible the relase of not more than %25 of the total amount of active substance in less than 2 hours in our extended release pharmaceutical tablet composition. As a result of this, we prevent the dose damping which is a disadvantage for the extended release dosage forms.

The present invention is also directed to a method of lowering blood glucose levels in human patients needing treatment for non-insulin dependent diabetes mellitus (NDDM), orally administering to human patients on a once-a-day basis a dose of a drug comprising a gliclazide or a pharmaceutically acceptable salt thereof, mannitol as a binder and hydrophilic diluent in a ratio of 5 to 15% (w/w) of total tablet composition that has a lower glysemic index, calcium hydrogen phosphate dihydrate or calcium hydrogen phosphate anhydrous in a ratio of 6 to 50 % (w/w) of total tablet composition and hydroxypropylmethylcellulose (HPMC), being comprised in an extended release pharmaceutical tablet composition; **characterised in that** does not comprise the glucose syrup.

In certain preferred embodiments, mannitol is used as a binder and hydrophilic diluent because it does not increase the blood glucose level in human patients by its low glysemic index activity.

In preferred embodiments, the extended release solid oral dosage form of the present invention is a pharmaceutical tablet composition which doesn't release more than %25 of the total amount of active substance in less than 2 hours, comprising: (a) a therapeutically effective amount of gliclazide or a pharmaceutically acceptable salt thereof (b) mannitol as a binder and hydrophilic diluent in a ratio of 5 to 15% (w/w) of total tablet composition that does not increase the blood glucose level in human patients (c) calcium hydrogen phosphate dihydrate or calcium hydrogen phosphate anhydrous as a diluent in a ratio of 6 to 50 % (w/w) of total tablet composition and (d) hydroxypropylmethylcellulose (HPMC) as a rate controlling polymer; said oral dosage form does not increase the blood glucose level; characterised in that does not comprise the glucose syrup.

In certain preferred embodiments, the extended release pharmaceutical tablet composition, wherein upon placement of the composition in an in vitro dissolution test comprising USP paddle method at 75 rpm in 900 ml media (pH 7.5 phosphate buffer) at 37° C, the percentages of HPMC with calcium hydrogen phosphate dihydrate or calcium hydrogen phosphate anhydrous and mannitol make possible the release of 0 to 25% of the total amount of gliclazide or a pharmaceutically acceptable salt thereof by a time of 2 hours and the release of 85% of the total amount of gliclazide or a pharmaceutically acceptable salt thereof by a time of 24 hours in the test.

By using mannitol in different rates with calcium hydrogen phosphate dihydrate or calcium hydrogen phosphate anhydrous we have surprisingly found that our extended release dosage form is much stabilised and we observed no change in our dissolution profile results till the end of the shelf-life.

Stability of pharmaceutical compositions may be affected by several factors, including the stability of the active pharmaceutical ingredient ("API"), API-excipient incompatibilities, and mode of packaging. Factors such as oxidation, moisture, heat and light may initiate and/or accelerate a chemical interaction, thereby degrading the API in a composition.

In certain preferred embodiments, the present invention relates to more stabilized extended release pharmaceutical tablet compositions of gliclazide or a pharmaceutically acceptable salt thereof in combination with mannitol in a ratio of 5 to 15% (w/w) of total tablet composition, calcium hydrogen phosphate dihydrate or calcium hydrogen phosphate anhydrous in a ratio of 6 to 50 % (w/w) of total tablet composition and hydroxypropylmethylcellulose (HPMC), to prevent the decomposition and thereby giving a stable composition with any individual impurity less than about 0.4% when stored at 25° C with a relative humidity of 60% or at accelerated conditions such as 40° C with a relative humidity of 75%.

As mentioned above, hydroxypropyl methylcellulose is 100 cP or 4000 cP or combination of these and is present in an amount of from 2 to 25%, calcium hydrogen phosphate dihydrate or calcium hydrogen phosphate anhydrous is present in an amount of from 6 to 50% and the mannitol is present in an amount of from 5 to 15%.

The problems associated with the prior art products are solved, and the above objectives of the invention are achieved by a novel composition and a method comprising an extended release pharmaceutical tablet composition.

The invention is further defined by reference to the following example. Although the example is not intended to limit the scope of the present invention, it should be considered in the light of the description detailed above.

### Example

Gliclazide extended release pharmaceutical tablet composition

This example describes the extended release pharmaceutical tablet composition containing gliclazide or a pharmaceutically acceptable salt thereof. Active ingredient and excipients of the tablet are given below:
Gliclazide 30 mg

### Extended release (XL) tablet

| Active ingredient | **Amount** |
|---|---|
| **Gliclazide** | 30.00 mg |
| Excipients | |
| Calcium hydrogen Phosphate Dihydrate | 74.88 mg |
| Hydroxypropyl Methylcellulose 4000 cP | 18.00 mg |
| Hydroxypropyl Methylcellulose 100 cP | 16.00 mg |
| Mannitol | 20.00 mg |
| Colloidal Silicon Dioxide | 0.32 mg |
| Magnesium Stearate | 0.80 mg |
| **Total** | **160 mg** |

## Claims

1. An extended release pharmaceutical tablet composition which does not release more than %25 of the total amount of active substance in less than 2 hours comprising :
a. a therapeutically effective amount of gliclazide or a pharmaceutically acceptable salt thereof
b. mannitol as a binder and hydrophilic diluent in a ratio of 5 to 15% (w/w) of total tablet composition that does not increase the blood glucose level of a human patient
c. calcium hydrogen phosphate dihydrate or calcium hydrogen phosphate anhydrous as a diluent in a ratio of 6 to 50% (w/w) of total tablet composition
d. and hydroxypropylmethylcellulose (HPMC), as a rate controlling polymer said oral dosage form does not increase the blood glucose level.

2. An extended release pharmaceutical tablet composition according to claim 1 where the rate controlling polymer is hydroxypropylmethylcellulose 100 cP or hydroxypropylmethylcellulose 4000 cP or combination of them.

3. An extended release pharmaceutical tablet composition according to claim 1 to 2 where the hydroxypropylmethylcellulose is present in an amount of from 2 to 25%.

4. The extended release pharmaceutical tablet composition according to claim 1 further comprising at least one additive ingredient.

5. The extended release pharmaceutical tablet composition according to claim 4 wherein said additive ingredient is selected from the group comprising of flow agents and lubricants.

6. An extended release pharmaceutical tablet composition according to claim 5 where the flow agent is selected from the group consisting of colloidal silicon dioxide, silica, calcium silicate, magnesium trisilicate, sodium stearyl fumarate and talc.

7. An extended release pharmaceutical tablet composition according to claim 5 and 6 where the flow agent is colloidal silicon dioxide.

8. An extended release pharmaceutical tablet composition according to claim 5, 6 and 7 where the flow agent is present in an amount from 0.25 to 2.0%.

9. An extended release pharmaceutical tablet composition according to claim 5 where the lubricant is selected from the group consisting of magnesium stearate, calcium stearate, stearic acid, hydrogenated vegetable oils, vegetable based fatty acids.

10. An extended release pharmaceutical tablet composition according to claim 5 and 9 where the lubricant is magnesium stearate.

11. An extended release pharmaceutical tablet composition according to claim 5, 9 and 10 where the lubricant is present in an amount of 0.25 to 2.0%.

12. An extended release pharmaceutical tablet composition according to claim 1, **characterised in that** the percentages of HPMC with calcium hydrogen phosphate dihydrate or calcium hydrogen phosphate anhydrous and mannitol make possible the release of 0 to 25% of the total amount of gliclazide or a pharmaceutically acceptable salt thereof by a time of 2 hours and the release of 85% of the total amount of gliclazide or a pharmaceutically acceptable salt thereof by a time of 24 hours.

13. An extended release pharmaceutical tablet composition according to claim 1, **characterised in that** does not comprise the glucose syrup.
